# EUROPEAN PATENT SPECIFICATION

(11) **EP 0 789 716 B1**
(45) Date of publication and mention of the grant of the patent: **26.04.2000**
(21) Application number: 95936566.9
(22) Date of filing: 30.10.1995
(51) Int. Cl.: C07K 16/18, C07K 16/30, A61K 39/395, G01N 33/53

(54) **ANTIBODIES AGAINST ALLOGENIC AND XENOGENIC PROTEINS, THEIR USE IN DIAGNOSIS AND THERAPY AND METHODS FOR THE DETERMINATION THEREOF**
ANTIKÖRPER GEGEN ALLOGENE UND XENOGENE PROTEINE, IHRE VERWENDUNG IN DIAGNOSE UND THERAPIE UND VERFAHREN ZU IHRER BESTIMMUNG
ANTICORPS DIRIGES CONTRE DES PROTEINES ALLOGENES ET XENOGENES, LEUR UTILISATION EN DIAGNOSTIC ET THERAPIE, AINSI QUE PROCEDES CONCERNANT LEUR DETERMINATION

(30) Priority: 04.11.1994 IT MI942238
(43) Date of publication of application: 20.08.1997
(73) Proprietor: ZETESIS S.P.A., 20122 Milano (IT)
(72) Inventor: BARTORELLI, Alberto, I-20145 Milano (IT)
(74) Representative: Minoja, Fabrizio, Dr.
(86) International application number: EP9504239
(87) International publication number: WO9614340

(56) References cited:
- WO-A-92/10197
- EUROPEAN JOURNAL OF BIOCHEMISTRY, vol. 212, no. 3, March 1993 pages 665-673, LEVY-FAVATIER ET AL 'CHARACTERIZATION,PURIFICATION AND CDNA CLONING OF A RAT PERCHLORIC-ACID-SOLUBLE 23-KDA PROTEIN PRESENT ONLY IN LIVER AND KIDNEY'

## Description

The present invention refers to diagnostic methods for the detection of natural antibodies recognizing proteins extractable from mammalian organs having molecular weight of about 14 Kda endowed with antitumoral activity when administered to xenogenic species.

The invention also refers to said natural antibodies in purified form and to their use in diagnostics and therapy.

W092/10197 discloses protein substances obtainable by extraction of mammalian organs, particularly liver, with perchloric acid and hypertonic saline solutions.

One of said substance having molecular weight of about 14 Kda has been isolated and partially sequenced (italian patent application no. MI 94001469) and turned out to be the main protein responsible of the observed biological properties, namely antitumor activity and ability to raise, when administered to animals of a different species from that from which it was extracted, antibodies able to recognize tumor antigens.

Said italian patent application also discloses two monoclonal antibodies, secreted by the hybridomas deposited at the European Collection of Animal Cell Cultures (ECACC), Porton Down, Salisbury, UK under the numbers 930806103 and 930806104, recognizing epitopes common to tumor antigens and to said 14 Kda protein from GOAT liver (hereinafter referred to as UK 114) used as antigen in the hybridoma preparation.

It has now been found that natural antibodies recognizing both allogenic and xenogenic proteins extractable from organs (particularly liver) endowed with the above described properties and present in about 93% of solid human tumors, circulate in mammalian serum. Said antibodies, normally present in low titer (< 1/100) in the serum of healthy animals or humans, may be stimulated by injecting the corresponding antigen, causing a remarkable increase of the titer and ability in sera to produce cytotoxic effects against cancer cells.

Independently from the possible interpretations of the observed results and of the meaning of the presence of natural anti-UK 114 antibodies, interpretations which are irrelevant for the validity of the present invention, said antibodies and their detections are useful for diagnostic, prognostic and therapeutic purposes.

In a first embodiment, the invention refers therefore to a method for the immunological detection of natural anti-UK 114 antibodies. The invention also concerns the purified anti-UK 114 antibodies and their use in diagnostics, therapy and immunocytochemistry. The methods for the detection of the natural antibodies are of conventional type and may rely on direct, competitive or "sandwich" type reactions. The protocols may use either solid supports or immunoprecipitation techniques. The antibodies and/or UK 114-type antigens used in the immunoassays will be suitably labelled with enzymes (for instance in the ELISA techniques) or with radioactive or fluorescent, compounds, dyes or pigments etc. The signal of the immunocomplex may optionally be enhanced by means of the biotin-avidin system.

The choice of the configuration of the method and of the relative materials is well within the skill of an average technician.

It has also been found that the anti-UK 114 antibodies present in the serum of patients treated with the proteins disclosed in W092/10197 or with purified UK 114 are endowed with in vitro and in vivo cytotoxic and cytolytic activity against human tumor cells. The invention, in a further embodiment, also provides a cytolytic and cytotoxic composition comprising monoclonal or polyclonal antibodies or hyperimmune sera, raised by immunizing animals or humans with proteins extractable from goat liver with perchloric acid and recognized by the monoclonal antibodies secreted by the hybridomas deposited at ECACC under accession numbers 930806103 or 930806104.

In vivo, the cytotoxic activity reaches a peak a few days after the antigen administration (UK 114 or UK 101) and slowly decreases in the subsequent weeks. The titer of anti-UK 114 antibodies of IgG type, by contrast, slowly increases in time, as evaluated by an ELISA method. It could be presumed, therefore, that the first peak of cytotoxicity is due to IgM-type antibodies, and/or to other serum components until now not identified.

It is anyhow evident the importance of methods allowing the determination of antibodies and/or cytotoxicity so that the therapy can be suitably monitored.

The administration of the antibodies, either natural or monoclonal, according to the invention may be carried out parenterally at doses sufficient to achieve a cytotoxic effect. The suitable administration methods are substantially identical with those already known for the administration of vaccines, antibodies or hyperimmune sera. This invention and the following examples 2-4 provide further guideline for the therapeutic and diagnostic applications of the above described antibodies.

The following Examples further illustrate the invention.

### Example 1

2,5% SDS and 5% mercaptoethanol were added to 100 µl of perchloric extract of goat liver (W092/10197; UK 101). The samples were heated to 100°C for 5 minutes amd 4 µl were analyzed in SDS-PAGE using, the PHAST SYSTEM apparatus (Pharmacia) with polyacrylamide 8/25 gradient gel (Pharmacia). Alternatively, homogeneous gels even at high density may be used. The separated proteins were electrophoretically transferred using the Phast-Transfer (Pharmacia) apparatus. The nitrocellulose membrane (0,22 µ) was previously equilibrated in Tris (25 mM) glycine (192 mM) buffer to which 20% of methanol was added. The run was performed at 5V x 20 min. (I A/cm²). The membrane was then incubated, after a saturation passage, to prevent aspecific binding, with the serum under examination.

The binding of the antibody optionally present is detected with a second anti-human A b, suitably labeled for colorimetric, radioimmunometric or chemiluminescent methods.

### Example 2

Enzyme-linked immunosorbent assays (ELISA) for the detection of antibodies to UK 114.

The following buffers were used:
(A) 50 mM sodium bicarbonate pH 8.5,
(B) 50 mM sodium phosphate pH 7.2,
(C) 50 mM sodium phosphate pH 7.4, 0.1% (w/v) NaN₃,
(D) 40 mM potassium phosphate pH 7,4, 150 mM NaCl, 0,02% (w/v) NaN₃, 0.05 (v/v) Tween 20,
(E) 50 mM sodium phosphate pH 7.4, 0.1% (w/v) NaN₃, 0.1% (w/v) BSA,
(F) 50 mM sodium phosphate pH 7.4, 150 mM NaCl, 0.1% (w/v) NaN₃, 0.5% (w/v) BSA protease-free, 0.05% (v/v) Tween 20, 0.%% (v/v) normal goat
3.5 ml of UK 114 (3.5 mg) in buffer A are mixed with 9 µl of NHS-LC Biotin (S.p.A., Milan) at 2 mg/ml in dimethyl sulfoxide. The mixture was incubated at 22°C for 3 hours and dialyzed for 24 hours at 4°C against several changes of buffer B in Spectrapor 6 dialysis tube (cut off 3500 D).

Microtiter wells were coated with 100 pl of BSA-biotin (5 µg/ml in buffer C) for 24 hours at 4°C, then washed and treated with 100 µl of streptavidin (5 µg/ml in buffer E). After a second overnight incubation at 4°C the wells were washed and treated with 100 µl of UK 114-biotin at different concentration (2, 1 and 0.5 µg/ml in buffer E) for 24 hours at 4°C. After washing the wells were blocked.

Uncoated antigen wells were made following the same procedure, except the third incubation step using buffer E instead of UK 114-biotin solution.

The washing step was performed by adding 300 µl/well of buffer D three times. The blocking was effected by adding 300 µl of buffer C containing 10% of sucrose and different blocking agents: 1% and 5% of bovine serum albumin, 1% of glycine, 1% of BSA added with 3% polyvinylpirrolidone, 5% of normal goat serum. After 30 minutes at room temperature blocking solution was discarded and the wells were dried in vacuum chamber and then stored wrapped in polyethylene and aluminium foil at 4°C until use.

### ELISA Procedure

Serum samples and negative control were diluted 1:21 with buffer F before being assayed. 100 µl of samples and standard solutions (1, 2, 4 and 8 U/mL in buffer F) were added to coated wells and incubated for 1 hour at room temperature on horizontal shaker (400-500 rpm). The wells were then washed four times with 300 µl of buffer D following which 100 µl of a 1:3000 solution of goat anti-human IgG horseradish peroxidase-labelled antibody dissolved in Stabilgen Buffer were added. After a 1 hour incubation at room temperature on horizontal shaker (400-500 rpm) the plates were washed and developed using 100 µl of Blue Star Solution (H₂O₂/TMB). After incubation at room temperature (18-24'C) for 15 min., the reaction was stopped by adding 100 µl of 1 M H₂SO₄, and the optical density (OD) was read using dual wavelenght photometer at 450 nm and 630 nm as the reference wavelength.

Specimens with OD higher than 8 U/mL standard were further diluted before being assayed.

The specimen concentration was calculated from the standard curve.

### Determination of Titer in Patients Undergoing Immunotherapy

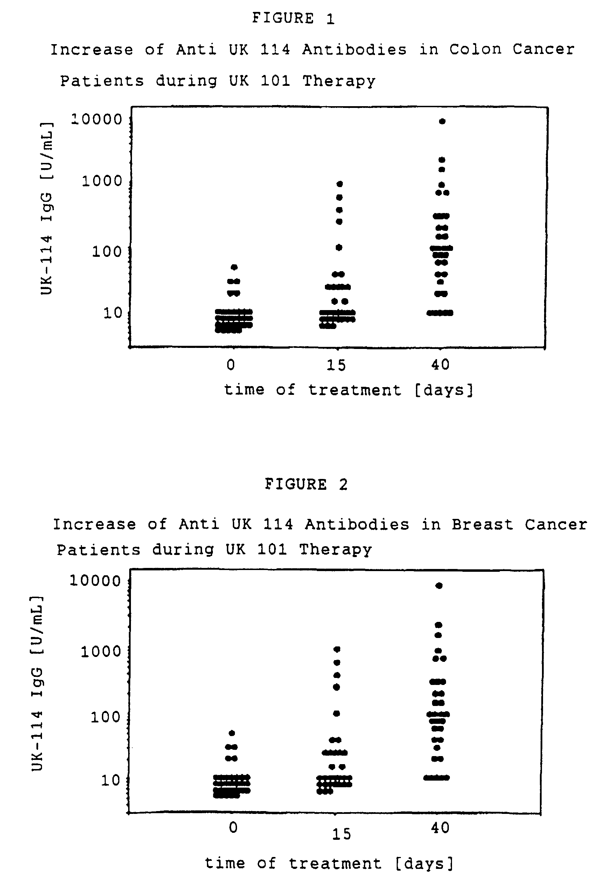

The evaluation of anti UK 114 antibody titre which is carried out in patients undergoing UK 101 therapy shows an antibody response higher than background value in 28% of breast cancer patients and 46% of colon cancer patients just after 15 days of UK 101 administration (4 subcutaneous injections). After 40 days from the beginning of UK 101 therapy, the increase of anti-UK 114 antibody titer can be detected in 63% and 97% of the colon and breast cancer patients, respectively (Figures 1 and 2).

### Example 3

Cytotoxic effect of anti-UK 114 antibodies on tumoral cell lines.

The KATO-III (gastric carcinoma) and the NICH (small cell lung carcinoma) tumoral cell lines, respectively positive and negative for surface staining by indirect immunofluorescence with the monoclonal antibody secreted from the hybridoma P3D1DII deposited at ECACC under n. 930806103 and rabbit polyclonal antibody anti-UK 114 (rAb), were used for the cytotoxic studies. The cytotoxicity was evaluated as percentage of ⁵¹Cr release from cell lines incubated for variable period of time with different concentration of mAb P3D1DII, Rb anti-UK 114 Ab or with an unrelated monoclonal or polyclonal antibody as control, in the presence of a source of heterologous (guinea pig) or homologous complement or in the presence of purified human monocytes. It was found that both mAb P3D1DII and anti-UK 114 rAb but not the unrelated control antibodies induced in the KATO-III a complement-dependent cytotoxicity, which was absent when heat inactivated or C9 deficient sera were used as a source of complement. Cytotoxicity was restored when C9 deficient serum was reconstituted with purified C9. Moreover, the Rb anti-UK 114 Ab but not the unrelated Rb Ab induced antibody dependant cellular cytotoxicity (ADCC) when KATO-III were used as target. Preabsorption of anti UK-114 Ab with the purified antigen abolished the antibody-induced cytotoxicity. In contrast, no complement cytotoxicity or ADCC was observed for NICH. These results suggest that anti-UK 114 antibodies have a potential complement and antibody-cell dependent cytotoxicity for the tumoral cell lines with the surface expression of UK 114 antigen.

### Example 4

In vivo cytotoxicity of anti-UK 114 antibodies on nude mice xenografted with human tumoral cell lines.

Nude mice were challenged s.c. with 1 x 10⁶ HT29 human colon adenocarcinoma cells. 8 hours later they received an injection at tumor site of either 0.2 ml of an anti-UK 114 rabbit hyperimmune (1/12 titer) serum raised by immunizing rabbits with UK 114. The treatment was repeated at the 7^{th}, 11^{th}, 14^{th}, 18^{th}, 21^{st}, 25^{th} day after challenge. Another group of mice was similarly treated with sera from tumor patients that clinically responded to UK-114 administration and produced anti-UK 114 Ab.

A control group received only the vehicle. The treated groups showed a statistically significant increase of life-span and decrease of tumor takes. The tumor latency was also remarkably prolonged in the treated groups in comparison with the control group. By way of example, the results obtained with rabbit antiserum and with human serum are reported in the following Table.

| Treatment | Tumor Takes | Latency (days) | Survival (days) |
|---|---|---|---|
| Control | 12/12 | 9 | 30 |
| Rabbit anti-UK 114 serum | 2/12 | 28 | 84 |
| Human anti-UK 114 serum | 5/20 | 23 | 67 |

## Claims

1. A method for the immunologic detection of natural antibodies recognizing a protein extractable from mammalian organs recognized by the monoclonal antibodies secreted by the hybridomas deposited at ECACC under accession numbers 930806103 or 930806104 and present in about 93% of human solid tumors comprising the detection of an immunocomplex between said natural antibodies and said optionally labelled proteins.

2. A method according to claim 1 wherein the immunocomplex is detected with a second labelled anti-species antibody.

3. Natural antibodies recognizing proteins extractable from mammalian organs recognized by the monoclonal antibodies secreted by the hybridomas deposited at ECACC under accession numbers 930806103 or 930806104 and present in about 93% of human solid tumors.

4. Cytotoxic compositions comprising antibodies or hyperimmune sera, raised by immunizing animals or humans with proteins extractable from goat liver with perchloric acid and recognized by the monoclonal antibodies secreted by the hybridomas deposited at ECACC under accession numbers 930806103 or 930806104.

## Patentansprüche

1. Verfahren zum immunologischen Nachweis natürlicher Antikörper, die ein aus Säugerorganen extrahierbares, durch die von Hybridoma, die beim ECACC unter den Zugangsnummern 930806103 oder 930806104 hinterlegt sind, abgesonderten, monoklonalen Antikörper erkanntes Protein erkennen und in etwa 93 % der festen Tumoren beim Menschen vorliegen, das den Nachweis eines Immunkomplexes zwischen diesen natürlichen Antikörpern und diesen gegebenenfalls markierten Proteinen umfaßt.

2. Verfahren nach Anspruch 1, wobei der Immunkomplex mit einem zweiten markierten Antispezies-Antikörper nachgewiesen wird.

3. Natürliche Antikörper, die aus Säugerorganen extrahierbare, durch die von Hybridoma, die beim ECACC unter den Zugangsnummern 930806103 oder 930806104 hinterlegt sind, abgesonderten, monoklonalen Antikörper erkannte Proteine erkennen und in etwa 93 % der festen Tumoren beim Menschen vorliegen.

4. Zytotoxische Mittel, die Antikörper oder hyperimmune Seren umfassen, gebildet durch Immunisieren von Tieren oder Menschen mit Proteinen, die mit Perchlorsäure aus Ziegenleber extrahierbar sind und von den beim ECACC unter den Zugangsnummern 930806103 oder 930806104 hinterlegten Hybridoma abgesonderten monoklonalen Antikörpern erkannt werden.

## Revendications

1. Procédé pour la détection immunologique d'anticorps naturels reconnaissant une protéine pouvant être extraite d'organes de mammifère, reconnue par les anticorps monoclonaux sécrétés par les hybridomes déposés à l'ECACC sous l'un des numéros d'accès 930806103 et 930806104 et présente dans environ 93 % des tumeurs solides humaines, comprenant la détection d'un complexe immunologique entre lesdits anticorps naturels et lesdites protéines éventuellement marquées.

2. Procédé selon la revendication 1, dans lequel le complexe immunologique est détecté avec un deuxième anticorps marqué anti-espèce.

3. Anticorps naturels reconnaissant des protéines pouvant être extraites d'organes de mammifère, reconnues par les anticorps monoclonaux sécrétés par les hybridomes déposés à l'ECACC sous l'un des numéros d'accès 930806103 et 930806104 et présentes dans environ 93 % des tumeurs solides humaines.

4. Compositions cytotoxiques comprenant des anticorps ou un sérum hyperimmun, générés par immunisation d'animaux ou d'êtres humains par des protéines pouvant être extraites de foie de chèvre avec de l'acide perchlorique et reconnues par les anticorps monoclonaux sécrétés par les hybridomes déposés à l'ECACC sous l'un des numéros d'accès 930806103 et 930806104.
